# FASCICULE DE BREVET EUROPEEN

(11) **EP 2 792 715 B1**
(45) Date de publication et mention de la délivrance du brevet: **16.10.2019**
(21) Numéro de dépôt: 14162779.4
(22) Date de dépôt: 31.03.2014
(51) Int. Cl.: C08L 91/06, A61K 47/06, A61K 8/31, C10M 101/02, A61Q 19/00, C08L 91/00

(54) **Vaselines semi-synthétiques ou synthétiques**
Halbsynthetische oder synthetische Vaselinetypen
Semi-synthetic or synthetic petroleum jelly

(30) Priorité: 15.04.2013 FR 1353396
(43) Date de publication de la demande: 22.10.2014
(73) Titulaire: Aiglon, 60460 Precy Sur Oise (FR)
(72) Inventeur: Puyoo, Jean-Jacques, 75008 PARIS (FR)
(74) Mandataire: Regimbeau

(56) Documents cités:
- WO-A2-2013/080138
- US-A1- 2006 057 168
- M. BEKKER ET AL: "The benefits of Fischer-Tropsch waxes in synthetic petroleum jelly", INTERNATIONAL JOURNAL OF COSMETIC SCIENCE, vol. 35, no. 1, 27 février 2013 (2013-02-27), pages 99-104, XP055076224, ISSN: 0142-5463, DOI: 10.1111/ics.12011

## Description

La présente invention concerne des vaselines semi-synthétiques ou synthétiques comprenant au moins 40 % en poids de matière première non issue du raffinage du pétrole.

Connue depuis plus de 100 ans, la vaseline, appelée en anglais « petroleum jelly », est un équilibre subtil d'hydrocarbures liquides et solides.
Le rôle des hydrocarbures solides semi-amorphes est de retenir dans un maillage fibreux suffisamment dense, des hydrocarbures huileux de poids moléculaire généralement élevé. L'ensemble peut être assimilé à un gel colloïdal amorphe comprenant deux phases : une phase continue constituée par la cire solide contenant l'huile en dispersion qui constitue la seconde phase.
Cette structure plastique et semi-solide confère aux vaselines leur consistance pâteuse, leur onctuosité, leur texture fibreuse et leurs propriétés lubrifiantes.
Les éléments solides ou liquides des hydrocarbures sont généralement issus du raffinage du pétrole et peuvent comporter notamment de 16 à 60 atomes de carbone avec des poids moléculaires sensiblement différents ; les structures possibles sont donc très diverses et en quantité pratiquement infinie.

La vaseline occupe une place importante dans l'arsenal des ingrédients pharmaceutiques et cosmétiques. Sa nature chimique et sa microstructure colloïdale définissent ses qualités multiples, ainsi que ses utilisations en tant que :

### - Excipient neutre :

De la même façon que l'eau est utilisée comme véhicule inerte des substances actives hydrophiles dans les tests d'allergie cutanés (patch), la vaseline est communément employée pour véhiculer les substances actives lipophiles dans de nombreuses applications.
L'inertie étant une des qualités les plus recherchées pour les excipients, ceux-ci doivent être le plus neutre possible vis-à-vis de principes actifs fragiles comme les hormones, les vitamines, les antibiotiques, les parfums, les colorants, etc.

La vaseline est inerte ce qui implique une compatibilité totale quel que soit le principe actif. Les onguents à base de vaseline sont reconnus parmi les meilleurs systèmes de libération d'actifs en termes d'efficacité. Par exemple, l'efficacité des stéroïdes topiques peut être augmentée par l'application du film occlusif créé par l'onguent. En effet, la peau sous occlusion devient plus perméable et les principes actifs peuvent mieux pénétrer.
Cette inertie chimique et physiologique fait de la vaseline un véhicule idéal pour protéger les ingrédients actifs délicats, pour créer des pommades pour les peaux les plus sensibles, ou pour formuler les produits avec un pH acide ou alcalin (à titre d'exemple, crèmes dépilatoires, teintures pour les cheveux, crèmes à hautes doses d'alpha hydroxy acides pour les soins dermatologiques).

### - Agent structurant / facteur de consistance :

Grâce à sa consistance semi-solide, la vaseline peut être employée seule comme un agent structurant idéal pour les pommades hydrophobes.
La vaseline a la consistance et l'onctuosité des corps gras d'origine végétale ou animale, mais présente sur ces derniers l'avantage considérable d'être absolument neutre et inaltérable, de ne pas rancir et ne présenter ni goût ni odeur. En fonction de ses propriétés telles que la filance, la viscosité, le point de goutte ou la capacité d'absorber l'huile, elle peut être utilisée comme régulateur de consistance ou stabilisateur d'émulsion.

### - Agent protecteur et cicatrisant :

A la base, la vaseline pure est vendue en pharmacie notamment pour le traitement des coupures, brûlures, lésions d'irritation, pieds abîmés, et gerçures des lèvres, comme soin anti-froid, ou encore comme onguent pour soigner les fesses des nouveaux nés. Tous ces produits ont pour fonction première de couvrir et de protéger la peau des agressions mécaniques, thermiques, chimiques et bactériennes.
La vaseline est en effet imperméable à l'air et à l'eau, sa capacité d'étanchéisation des coupures et des brûlures empêchant l'humidité de la peau de s'évaporer.
Ainsi, elle fonctionne comme un écran qui protège la peau des agressions extérieures et permet son auto guérison en cas de lésions.

### - Agent hydratant :

En réduisant la perte d'eau à travers l'épiderme de plus de 98 %, la vaseline est un excellent hydratant occlusif de référence. Elle crée une barrière hydrophobe inerte à la surface de la peau, bloque la perte insensible en eau transcutanée et piège l'eau sous la surface de la peau.

Grâce à cet excellent pouvoir hydratant, de nombreux dermatologues utilisent la vaseline pour le traitement des peaux présentant des problèmes de sécheresse cutanée ou de squames. La vaseline accélère la recouvrance des propriétés normales de la barrière cutanée dans le cas de peau lésée. C'est sa raison d'être dans la composition des crèmes prescrites dans les cas de sécheresse cutanée, le psoriasis, la dermatite atopique, l'hyperkératose et les xérodermatoses.

### - Emollient :

Grâce à son pouvoir adoucissant, la vaseline est l'émollient par excellence. Ses propriétés non sensibilisantes et hautement occlusives sont efficaces contre la perte en eau transépidermique. Son rôle est de rester à l'extérieur de la peau : elle est donc très utile dans les crèmes cosmétiques protectrices, les produits de maquillage, les soins solaires et capillaires, et ceci pour toutes les ethnies, Des exemples de vaselines semi-synthétiques ou synthétiques sont divulgués dans l'article "The benefits of Fischer-Tropsch waxes in synthetic petroleum jelly", INTERNATIONAL JOURNAL OF COSMETIC SCIENCE, vol. 35, no. 1, 27 février 2013 (2013-02-27), pages 99-104. Ce document décrit des vaselines comprenant :
- 20% de cire microcristalline (minérale ou synthétique),
- 55 % de cire synthétique à bas point de fusion (obtenue par un procédé de Fischer Tropsch), et
- 25 % de paraffine synthétique.

Le procédé originel pour préparer des vaselines implique le raffinage et la rectification d'un produit de la distillation du pétrole brut. Les méthodes de raffinage varient suivant le type de pétrole brut utilisé et le procédé général de raffinerie. Avec ce type de procédé, on peut obtenir des produits de qualité très variable en fonction du degré de purification. Ce procédé a ses limites et, en tout état de cause, ne permet jamais de satisfaire aux exigences de la pharmacopée française Codex. De telles vaselines directement issues du pétrole sont appelées « petrolatum » en anglais.

Il est également possible de préparer des vaselines par mélange, c'est-à-dire par mélange de produits constitutifs des vaselines, à savoir des paraffines, des huiles blanches minérales et des cires microcristallines, ce procédé par mélange permettant d'obtenir les vaselines les plus pures avec une meilleure reproductibilité de la composition. En choisissant des matières premières (huiles blanches minérales, cires microcristallines et paraffines) hautement raffinées, de telles vaselines ont une pureté et une stabilité supérieures qui leur permettent de satisfaire les exigences de la pharmacopée française Codex.

Toutefois, les produits de base utilisés pour préparer les vaselines par mélange sont également issus du raffinage du pétrole. Les ressources en pétrole tendant à s'épuiser et les consommateurs souhaitant de plus en plus utiliser des produits ne dérivant pas du pétrole, il existe un réel besoin de pouvoir préparer des vaselines semi-synthétiques ou synthétiques, c'est-à-dire ne dérivant que partiellement ou pas du tout du raffinage du pétrole.

Une telle vaseline semi-synthétique ou synthétique pourrait en particulier être préparée par mélange en utilisant au moins en partie des matières premières synthétiques, et en particulier en remplaçant l'huile blanche minérale (encore appelée paraffine liquide ou huile de paraffine et Parrafinum liquidum selon la nomenclature INCI - « International Nomenclature of Cosmetic Ingrédients » - par exemple N° CAS 8002-74-2), qui représente au moins la moitié de la composition d'une vaseline dite par mélange, par une huile synthétique.

Toutefois, le changement d'une telle huile est très délicat car il faut trouver une huile qui permette de préparer une vaseline qui, d'une part, conservera les mêmes propriétés physico-chimiques que la vaseline préparée à partir de matières premières issues du raffinage du pétrole, et en particulier conservera sa texture, ses qualités sensorielles (odeur, couleur, brillance, etc.), ses qualités physiques (point de goutte, pénétration, viscosité, etc.), sa pureté, etc., et, d'autre part, satisfera toujours les exigences de la pharmacopée, notamment de la pharmacopée européenne et en particulier de la pharmacopée française Codex qui permet une utilisation orale de la vaseline.

Les inventeurs ont ainsi découvert qu'il était possible de préparer des vaselines par mélange en remplaçant l'huile blanche minérale par de l'huile blanche GTL, tout en conservant les mêmes propriétés physico-chimiques et en satisfaisant les exigences de la pharmacopée européenne et de la pharmacopée française Codex.

La présente invention a donc pour objet une vaseline comprenant, par rapport au poids total de la vaseline :
- 40 à 90 % en poids d'huile blanche GTL,
- 5 à 50 % en poids de cire micro cristalline, et
- 0 à 50 % en poids de paraffine.

### Huile blanche GTL :

Par « huile blanche GTL », on entend, au sens de la présente invention, une huile obtenue à partir d'un gaz (procédé dit « Gaz to Liquid »), en particulier par un procédé de Fischer-Tropsch permettant la synthèse d'hydrocarbures synthétiques par hydrogénation du monoxyde de carbone, éventuellement suivi d'une ou plusieurs étapes de traitement additionnel choisies notamment parmi un hydrocraquage, un déparaffinage et une hydrofinition (encore appelée hydroraffinage).

Il s'agira en particulier d'une isoparaffine en C₁₈-C₅₀ (C18-C50 Isoparaffin selon la nomenclature INCI), consistant majoritairement en des hydrocarbures saturés linéaires, cycliques et ramifiés en C₁₈-C₅₀.

De préférence, cette huile sera de qualité cosmétique ou pharmaceutique. En particulier, une telle huile sera conforme à la pharmacopée, et plus particulièrement à la pharmacopée européenne 7.0 01/2008:0239.

En particulier, la teneur en hydrocarbures aromatiques polycycliques (HAP) sera de préférence conforme à la pharmacopée européenne 7.0 01/2008:0239, de tels composés ayant un fort effet comédogène.
La vérification que la teneur en HAP est conforme à la pharmacopée européenne 7.0 01/2008:0239 est effectuée selon le protocole ci-dessous, en utilisant des réactifs pour spectrométrie dans l'ultraviolet :
*Introduire 25,0 mL de l'huile à tester dans une ampoule à décanter de 125 mL dont les parties en verre rodé ne sont pas lubrifiées (bouchon, robinet). Ajouter 25 mL d'hexane R préalablement lavé par agitation avec 2 fois 10 mL de diméthylsulfoxyde R. Mélanger et ajouter 5,0 mL de diméthylsulfoxyde R. Agiter vigoureusement pendant 1 min et laisser reposer jusqu'à formation de 2 phases limpides. Transférer la phase inférieure dans une 2^{nde} ampoule à décanter, ajouter 2 mL d'hexane R et agiter le mélange vigoureusement. Laisser reposer jusqu'à formation de 2 phases limpides. Séparer la phase inférieure et mesurer son absorbance (2.2.25) entre 260 nm et 420 nm, en utilisant comme liquide de compensation la phase inférieure limpide obtenue en agitant vigoureusement 5,0 mL de diméthylsulfoxyde R avec 25 mL d'hexane R pendant 1 min. Préparer une solution témoin contenant 7.0 mg*/*L de naphtalène R dans le triméthylpentane R et mesurer l'absorbance de cette solution à l'absorption maximum à 275 nm, en utilisant le triméthylpentane R comme liquide de compensation. A aucune longueur d'ondes entre 260 nm et 420 nm, l'absorbance de la solution à tester ne doit dépasser 1*/*3 de l'absorbance de la solution témoin mesurée à 275 nm.*

De même, la teneur en substances facilement carbonisables sera de préférence conforme à la pharmacopée européenne 7.0 01/2008:0239.
La vérification que la teneur en substances facilement carbonisables est conforme à la pharmacopée européenne 7.0 01/2008:0239 est effectuée selon le protocole ci-dessous : *Dans un tube à col rodé d'environ 125 mm de long et 18 mm de diamètre interne, gradué à 5 mL et 10 mL, lavé avec de l'eau R chaude (température d'au moins 60°C), de l'acétone R, de l'heptane R et finalement de l'acétone R, séché à 100-110°C et refroidi dans un dessiccateur, introduire 5 mL de l'huile à tester et ajouter 5 mL d'acide sulfurique R1 dépourvu d'azote. Insérer le bouchon rodé et agiter aussi vigoureusement que possible, dans la direction longitudinale du tube, pendant 5 s. Desserrer le bouchon, placer immédiatement le tube dans un bain-marie, en évitant le contact du tube avec le fond ou les côtés du bain et chauffer pendant 10 min. Après 2 min, 4 min, 6 min et 8 min, retirer le tube du bain et agiter aussi vigoureusement que possible, dans la direction longitudinale du tube, pendant 5 s. A la fin des 10 minutes de chauffage, retirer le tube du bain-marie et laisser reposer pendant 10 min. Centrifuger à 2000 g pendant 5 min. La phase inférieure ne doit pas être plus intensément colorée (2.2.2, méthode 1) que le mélange de 0,5 mL d'une solution bleu primaire, 1,5 mL d'une solution rouge primaire, 3,0 mL d'une solution jaune primaire et 2 mL d'une solution d'acide chlorhydrique R à 10 g*/*L.*

Avantageusement, la vaseline selon la présente invention contiendra de 50 à 90 %, notamment de 50 à 80% en poids d'huile blanche GTL telle que définie ci-dessus, par rapport au poids total de la vaseline.

### Cire microcristalline :

La cire microcristalline, ou Cera microcristallina selon la nomenclature INCI (par exemple N° CAS 63231-60-7 ou 64742-60-5), est constituée majoritairement d'hydrocarbures aliphatiques saturés de haut poids moléculaire, en particulier au moins en C₂₅. Elle peut contenir de 0 à 30 % d'huile (mesuré selon la norme ASTM D721). Le point de fusion de la cire microcristalline (mesuré selon la norme ASTM D127) est généralement compris entre environ 60 et environ 100°C et la pénétration à l'aiguille (mesurée selon la norme ASTM D1321) est généralement comprise entre 1 et 80 %.

Elle pourra être issue du raffinage du pétrole ou être synthétique. La cire microcristalline synthétique pourra être en particulier un polymère synthétique à base de copolymère d'éthylène et de propylène ou une cire obtenue par un procédé de Fischer-Tropsch, éventuellement suivi d'une ou plusieurs étapes de traitement additionnel choisies notamment parmi un hydroraffinage, un hydrocraquage et une hydroisomérisation.

Elle pourra être conforme en particulier aux critères de pureté FDA CFR 172886 et/ou FDA CFR 1783710.

### Paraffine :

La paraffine, ou Paraffin selon la nomenclature INCI (par exemple N° CAS 8002-74-2 ou 64742-51-4), est une paraffine solide constituée majoritairement d'un mélange solide d'hydrocarbures saturés généralement linéaires, de préférence en C₂₀-C₄₀.
La paraffine est solide à température ambiante et aura un point de fusion (mesuré selon la norme ASTM D87) généralement compris entre environ 40°C et environ 70°C.

La paraffine peut être issue du raffinage du pétrole ou être synthétique. La paraffine synthétique pourra notamment être obtenue par un procédé de Fischer-Tropsch, par hydrogénation du monoxyde de carbone, éventuellement suivi d'une ou plusieurs étapes de traitement additionnel choisies notamment parmi un hydroraffinage et un hydrocraquage.

Elle pourra être conforme en particulier aux critères de pureté FDA CFR 172886 et/ou FDA CFR 1783710. Elle pourra être de qualité cosmétique ou pharmaceutique. En particulier, une telle paraffine sera conforme à la pharmacopée, et plus particulièrement à la pharmacopée européenne 7.0 01/2008:1034.

En particulier, la teneur en hydrocarbures aromatiques polycycliques (HAP) sera de préférence conforme à la pharmacopée européenne 7.0 01/2008:1034.
La vérification que la teneur en HAP est conforme à la pharmacopée européenne 7.0 01/2008:1034 est effectuée selon le protocole ci-dessous, en utilisant des réactifs pour spectrométrie dans l'ultraviolet :
*Dissoudre 0,50 g de paraffine à tester dans 25 mL d'heptane R et placer le mélange résultant dans une ampoule à décanter de 125 mL dont les parties en verre rodé ne sont pas lubrifiées (bouchon, robinet). Ajouter 5,0 mL de diméthylsulfoxyde R. Agiter vigoureusement pendant 1 min et laisser reposer jusqu'à formation de 2 phases limpides. Transférer la phase inférieure dans une 2^{nde} ampoule à décanter, ajouter 2 mL d'heptane R et agiter le mélange vigoureusement. Laisser reposer jusqu'à formation de 2 phases limpides. Séparer la phase inférieure et mesurer son absorbance (2.2.25) entre 260 nm et 420 nm, en utilisant comme liquide de compensation la phase inférieure limpide obtenue en agitant vigoureusement 5,0 mL de diméthylsulfoxyde R avec 25 mL d'heptane R pendant 1 min. Préparer une solution témoin contenant 7.0 mg*/*L de naphtalène R dans le diméthylsulfoxyde R et mesurer l'absorbance de cette solution à l'absorption maximum à 278 nm, en utilisant le diméthylsulfoxyde R comme liquide de compensation. A aucune longueur d'ondes entre 260 nm et 420 nm, l'absorbance de la solution à tester ne doit dépasser 1*/*3 de l'absorbance de la solution témoin mesurée à 278 nm.*

Avantageusement, la vaseline selon la présente invention contiendra de 0 à 40 %, notamment de 0 à 20 % en poids de paraffine, par rapport au poids total de la vaseline.

Selon un mode de réalisation particulier, la vaseline selon l'invention comprend, par rapport au poids total de la vaseline :
- 50 à 90 % en poids d'huile blanche GTL, et en particulier d'isoparaffine en C₁₈ à C₅₀,
- 5 à 50 % en poids de cire micro cristalline, et
- 0 à 40 % en poids de paraffine,
notamment :
- 50 à 80 % en poids d'huile blanche GTL, et en particulier d'isoparaffine en C₁₈ à C₅₀,
- 5 à 50 % en poids de cire microcristalline, et
- 0 à 20 % en poids de paraffine.

Selon un autre mode de réalisation, la vaseline selon l'invention comprend, par rapport au poids total de la vaseline (correspondant à une vaseline dite standard) :
- 55 à 70 %, notamment 60 à 70 %, en poids d'huile blanche GTL, et en particulier d'isoparaffine en C₁₈ à C₅₀,
- 15 à 30 %, notamment 20 à 25 %, en poids de cire microcristalline, et
- 5 à 15 % en poids de paraffine.

Selon encore un autre mode de réalisation, la vaseline selon l'invention comprend, par rapport au poids total de la vaseline (correspondant à une vaseline dite fluide) :
- 70 à 80 % en poids d'huile blanche GTL, et en particulier d'isoparaffine en C₁₈ à C₅₀,
- 5 à 15 % en poids de cire micro cristalline, et
- 10 à 20 % en poids de paraffine.

Selon encore un autre mode de réalisation, la vaseline selon l'invention comprend, par rapport au poids total de la vaseline (correspondant à une vaseline dite filante) :
- 50 à 60 % en poids d'huile blanche GTL, et en particulier d'isoparaffine en C₁₈ à C₅₀, et
- 40 à 50 % en poids de cire microcristalline.

Selon encore un autre mode de réalisation, la vaseline selon l'invention comprend, par rapport au poids total de la vaseline :
- 40 à 50 % en poids d'huile blanche GTL, et en particulier d'isoparaffine en C₁₈ à C₅₀,
- 10 à 20 % en poids de cire microcristalline, et
- 40 à 50 % en poids de paraffine.

La vaseline selon l'invention satisfera en particulier les exigences de la pharmacopée, et plus particulièrement de la pharmacopée européenne 7.0 07/2009:1799 et de la pharmacopée française Codex de 2004.

En particulier, la teneur en hydrocarbures aromatiques polycycliques (HAP) sera de préférence conforme à la pharmacopée européenne 7.0 07/2009:1799.
La vérification que la teneur en HAP est conforme à la pharmacopée européenne 7.0 07/2009:1799 est effectuée selon le protocole ci-dessous, en utilisant des réactifs pour spectrométrie dans l'ultraviolet :
*Dissoudre 1,0 g de la vaseline à tester dans 50 mL d'hexane R préalablement lavé par agitation avec 2 fois 10 mL de diméthylsulfoxyde R. Transférer cette solution dans une ampoule à décanter de 125 mL dont les parties en verre rodé ne sont pas lubrifiées (bouchon, robinet). Ajouter 20 mL de diméthylsulfoxyde R. Agiter vigoureusement pendant 1 min et laisser reposer jusqu'à formation de 2 phases limpides. Transférer la phase inférieure dans une 2^{nde} ampoule à décanter. Répéter l'extraction avec 20 mL de diméthylsulfoxyde R. Agiter vigoureusement les phases inférieures réunies avec 20 mL d'hexane R pendant 1 min. Laisser reposer jusqu'à formation de 2 phases limpides. Séparer la phase inférieure et compléter à 50,0 mL avec du diméthylsulfoxyde R. Mesurer l'absorbance (2.2.25) entre 260 nm et 420 nm, sous une épaisseur de 4 cm et en utilisant comme liquide de compensation la phase inférieure limpide obtenue en agitant vigoureusement 10 mL de diméthylsulfoxyde R avec 25 mL d'hexane R pendant 1 min. Préparer une solution témoin contenant 6.0 mg de naphtalène R par litre dans du diméthylsulfoxyde R et mesurer l'absorbance de cette solution au maximum à 278 nm, sous une épaisseur de 4 cm et en utilisant le diméthylsulfoxyde R comme liquide de compensation. A aucune longueur d'ondes entre 260 nm et 420 nm, l'absorbance de la solution à tester ne doit dépasser l'absorbance de la solution témoin mesurée à 278 nm.*

De même, la teneur en matières carbonisables sera de préférence conforme à la pharmacopée française Codex de 2004.
La vérification que la teneur en matières carbonisables est conforme à la pharmacopée française Codex de 2004 est effectuée selon le protocole ci-dessous :
*Dans une éprouvette à col rodé, introduire 0,5 g de vaseline à tester. Ajouter 20,0 mL d'acide sulfurique R. Maintenir au bain-marie, pendant 10 min, en agitant pendant 5 s toutes les 2 min. Refroidir puis transvaser le contenu de l'éprouvette dans une ampoule à décanter parfaitement sèche. Laisser reposer pendant 10 min. Recueillir la phase inférieure, filtrer si nécessaire sur verre frité (4). Mesurer l'absorbance de la solution de 400 nm à 450 nm (2.2.25) en utilisant l'acide sulfurique R comme liquide de compensation. L'absorbance ne doit pas être supérieure à 0,40.*

La présente invention sera mieux comprise à la lumière des exemples non limitatifs qui suivent.

### EXEMPLES

Les huiles blanches GTL suivantes ont été utilisées dans les exemples de la présente invention :

| **Caractéristiques** | **Huile blanche GTL (1)** | **Huile blanche GTL (2)** | **Huile blanche GTL (3)** | **Huile blanche GTL (4)** |
|---|---|---|---|---|
| Nom chimique | Distillat lourd (Fischer Tropsch)-hydrocarbures linéaires cycliques et ramifies en C₁₈-C₅₀ | Distillat lourd (Fischer Tropsch)-hydrocarbures linéaires cycliques et ramifies en C₁₈-C₅₀ | Distillat lourd (Fischer Tropsch)-hydrocarbures linéaires cycliques et ramifies en C₁₈-C₅₀ | Distillat lourd (Fischer Tropsch)-hydrocarbures linéaires cycliques et ramifies en C₁₈-C₅₀ |
| Couleur Saybolt, ASTM D156 | + 30 | + 30 | + 30 | + 30 |
| Densité à 15°C, ISO12185 | 0,818 | 0,806 | 0,828 | 0,818 |
| Indice de Réfraction à 20°C, ASTM D1218 | 1,454 | 1,450 | 1,460 | 1,454 |
| Point d'éclair, ISO2592 | 225°C | 200°C | 266°C | 225°C |
| Point d'écoulement, ISO3016 | -36°C | -39°C | -24°C | -36°C |
| Viscosité cinématique, ISO3104 | 40 mm²/s (à 20°C) | 18 mm²/s (à 20°C) | 111 mm²/s (à 20°C) | 40 mm²/s (à 20°C) |
| | 18.5 mm²/s (à 40°C) | 9.3 mm2/s (à 40°C) | 43 mm²/s (à 40°C) | 18.5 mm²/s (à 40°C) |
| | 4.1 mm²/s (à 100°C) | 2.6 mm²/s (à 100°C) | 7.6 mm²/s (à 100°C) | 4.1 mm²/s (à 100°C) |
| Poids moléculaire, ASTM D2502 | 341 | 312 | 545 | 341 |
| Distillation, DIN EN 3405 | T°I: 398°C | T°I: 327°C | T°I: 462°C | T°I: 398°C |
| | T°F : 472°C | T°F : 374°C | T°F : 553°C | T°F : 472°C |
| Chaine de carbones | C₂₅-C₃₄ | C₂₀-C₂₇ | C₃₁-C₄₇ | C₂₅-C₃₄ |
| Teneur HAP, Ph. Eur.* | conforme (0,026 v. 0,099 pour le témoin) | Non réalisé | conforme (0,019 v. 0,099 pour le témoin) | conforme (0,006 v. 0,099 pour le témoin) |
| Substances facilement carbonisables, Ph. Eur.* | non-conforme (coloration supérieure au témoin) | non-conforme (coloration supérieure au témoin) | non-conforme (coloration supérieure au témoin) | conforme |
| | D.O maxi : 3,118 à 450nm | D.O maxi : 3,613 à 450nm | D.O maxi : 2,290 à 450nm | |

| | | | | |
|---|---|---|---|---|
| * Ph. Eur. = Pharmacopée Européenne 7.0 01/2008:0239 | | | | |

Les vaselines (1) à (4) ont été préparées à partir des huiles blanches GTL (1) à (4) décrites ci-dessus avec la formulation suivante :
- 64 % en poids d'huile blanche GTL,
- 26 % en poids de cire microcristalline, et
- 10 % en poids de paraffine.

Ces vaselines ont été comparées à une vaseline comparative de l'art antérieur comprenant de l'huile blanche minérale selon la formulation suivante :
- 65 % en poids d'huile blanche minérale,
- 25 % en poids de cire micro cristalline, et
- 10 % en poids de paraffine.

Les résultats suivants ont été obtenus :

| **Caractéristiques** | **Vaseline (1)** | **Vaseline (2)** | **Vaseline (3)** | **Vaseline (4)** | **Vaseline comparative** |
|---|---|---|---|---|---|
| Couleur | blanche | blanche | blanche | blanche | blanche |
| Odeur | néant | néant | néant | néant | néant |
| Goût | néant | néant | néant | néant | néant |
| Point de goutte, Ph. Eur.* | 55.3 °C | 64.2°C | 68 °C | 58,2°C | 55 °C |
| Pénétrabilité au cône, Ph. Eur.* (consistance) | 181 | 201 | 140 | 180 | 180-240 |
| Teneur HAP, Ph. Eur.* | conforme | conforme | conforme | conforme | conforme |
| Matières carbonisables, Ph. Fr.** | conforme | conforme | conforme | conforme | conforme |

| | | | | | |
|---|---|---|---|---|---|
| * Ph. Eur. = Pharmacopée Européenne 7.0 07/2009:1799 ** Ph. Fr.= Pharmacopée Française Codex de 2004 | | | | | |

Des tests sensoriels comparatifs ont également été réalisés sur les 4 types de vaseline suivants, comprenant soit de l'huile blanche GTL selon l'invention, soit de l'huile blanche minérale selon l'art antérieur :

**■ vaselines de type A (vaselines standard, base crème) :**

| Vaseline A minérale (**A m**) | Vaseline A base GTL (**A gtl**) |
|---|---|
| 64 % en poids d'huile blanche minérale | 66 % en poids d'huile blanche GTL(1) |
| 26 % en poids de cire microcristalline | 24 % en poids de cire microcristalline |
| 10 % en poids de paraffine | 10 % en poids de paraffine |

**■ vaselines de type B (vaselines standard avec un point de goutte élevé, base crème) :**

| **Vaseline** B minérale (**B m**) | **Vaseline** B base GTL (**B gtl**) |
|---|---|
| 67,5 % en poids d'huile blanche minérale | 67,5 % en poids d'huile blanche GTL(1) |
| 22,5 % en poids de cire microcristalline | 22,5 % en poids de cire microcristalline |
| 10 % en poids de paraffine | 10 % en poids de paraffine |

**■ vaselines de type C (vaseline fluide, base démaquillante) :**

| Vaseline C minérale (**C m**) | Vaseline C base GTL (**C gtl**) |
|---|---|
| 78 % en poids d'huile blanche minérale | 76 % en poids d'huile blanche GTL(1) |
| 4,5 % en poids de cire microcristalline | 5,5 % en poids de cire microcristalline |
| 17,5 % en poids de paraffine | 18,5 % en poids de paraffine |

**■ vaseline de type D (vaseline filante) :**

| Vaseline D minérale (**D m**) | Vaseline D base GTL (**D gtl**) |
|---|---|
| 55 % en poids d'huile blanche minérale | 53 % en poids d'huile blanche GTL(3) |
| 45 % en poids de cire microcristalline | 47 % en poids de cire microcristalline |

L'ensemble des vaselines mentionnées ci-dessus satisfont les exigences de la pharmacopée européenne 7.0 07/2009:1799 et de la pharmacopée française Codex de 2004.

L'ensemble de ces vaselines a été testé en aveugle par 5 personnes afin de comparer les caractéristiques sensorielles d'une vaseline selon l'invention par rapport à une vaseline minérale selon l'art antérieure.

9 critères ont été examinés, à savoir l'odeur, le filant, la texture, la brillance, la brillance sur la peau, l'étalement, le collant, la lubrification et l'absorption des vaselines.

Les résultats obtenus avec chacune des 5 personnes sont présentés dans le tableau ci-dessous.

| **Vaseline** | **A m** | **A gtl** | **B m** | **B gtl** | **C m** | **C gtl** | **D m** | **D gtl** |
|---|---|---|---|---|---|---|---|---|
| **Odeur** | 5* | 5= | 5* | 5= | 5* | 5= | 5* | 5= |
| **Filant** | 3* | 5= | 5** | 4= | 4* | 4= | 5*** | 5= |
| | 2** | | | 1- | 1ø | 1- | | |
| **Texture** | 3** | 5= | 3** | 4= | 1** | 5= | 1** | 5= |
| | 2*** | | 2*** | 1+ | 4*** | | 4*** | |
| **Brillance** | 4** | 4- | 1* | 5= | 1* | 5= | 2* | 5= |
| | 1*** | 1= | 4** | | 2** | | 3** | |
| | | | | | 2*** | | | |
| **Brillance sur la peau** | 5** | 3= | 1* | 5= | 5*** | 4= | 4* | 5= |
| | | 2- | 4** | | | 1+ | 1*** | |
| **Etalement** | 4** | 5= | 4** | 5= | 1** | 5= | 3* | 5= |
| | 1*** | | 1*** | | 4*** | | 2** | |
| **Collant** | 4* | 5= | 5** | 3= | 5* | 4= | 2** | 5= |
| | 1** | | | 2- | | 1+ | 3*** | |
| **Lubrification** | 3** | 4= | 4** | 3= | 1** | 5= | 3* | 5= |
| | 2*** | 1- | 1*** | 1+ | 4*** | | 2** | |
| | | | | 1- | | | | |
| **Absorption** | 1*4** | 3= | 3* | 2= | 1* | 4= | 1* | 5= |
| | | 2- | 1** | 3+ | 2** | 1- | 4** | |
| | | | 1*** | | 2*** | | | |

| | | | | | | | | |
|---|---|---|---|---|---|---|---|---|
| Odeur : * pas d'odeur ; ** odeur moyenne ; *** odeur forte Filant : ø non filant ; * fibre courte ; ** fibre moyenne ; *** fibre longue Texture : * granuleux ; ** moyenne ; *** très souple Brillance : * mate ; ** moyenne ; *** très brillante Etalement : * aucun ; ** difficile ; *** moyen ; **** excellent Collant : * pas collant ; ** légèrement collant ; *** très collant Lubrification : ø aucune ; * glisse peu ; ** glisse moyennement ; *** glisse bien Absorption : * lente ; ** moyenne ; *** rapide | | | | | | | | |

Pour les résultats obtenus avec les vaselines selon l'invention, le signe « = » signifie que la personne n'a pas perçu de différence avec la vaseline minérale correspondante, le signe « + » signifie que la personne trouve la vaseline selon l'invention meilleure que la vaseline minérale correspondante et le signe « - » signifie que la personne trouve la vaseline selon l'invention moins bonne que la vaseline minérale correspondante.

L'ensemble de ces résultats montrent que les vaselines préparées à partir d'huile blanche GTL ont des caractéristiques similaires aux vaselines préparées à partir d'huile blanche minérale et sont conformes à la pharmacopée, en particulier à la pharmacopée européenne et à la pharmacopée française.

## Revendications

1. Vaseline comprenant, par rapport au poids total de la vaseline :
- 40 à 90 % en poids d'huile blanche GTL,
- 5 à 50 % en poids de cire microcristalline, et
- 0 à 50 % en poids de paraffine.

2. Vaseline selon la revendication 1, **caractérisée en ce que** l'huile blanche GTL est préparée par un procédé Fischer-Tropsch, éventuellement suivi d'une ou plusieurs étapes de traitement choisies parmi un hydrocraquage, un déparaffinage et une hydrofinition.

3. Vaseline selon la revendication 1 ou 2, **caractérisée en ce que** l'huile blanche GTL est une isoparaffine en C₁₈-C₅₀.

4. Vaseline selon l'une quelconque des revendications 1 à 3, **caractérisée en ce que** la teneur en hydrocarbures aromatiques polycycliques (HAP) de l'huile blanche GTL est conforme à la pharmacopée européenne 7.0 01/2008:0239.

5. Vaseline selon l'une quelconque des revendications 1 à 4, **caractérisée en ce que** la teneur en substances facilement carbonisables de l'huile blanche GTL est conforme à la pharmacopée européenne 7.0 01/2008:0239.

6. Vaseline selon l'une quelconque des revendications 1 à 5, **caractérisée en ce que** l'huile blanche GTL est conforme à la pharmacopée européenne 7.0 01/2008:0239.

7. Vaseline selon l'une quelconque des revendications 1 à 6, **caractérisée en ce que** la cire microcristalline est issue du raffinage du pétrole ou est synthétique.

8. Vaseline selon la revendication 7, **caractérisée en ce que** la cire microcristalline synthétique est un polymère synthétique à base de copolymère d'éthylène et de propylène ou une cire obtenue par un procédé de Fischer-Tropsch, éventuellement suivi d'une ou plusieurs étapes de traitement choisies parmi un hydroraffinage, un hydrocraquage et une hydroisomérisation.

9. Vaseline selon l'une quelconque des revendications 1 à 8, **caractérisée en ce que** la paraffine est issue du raffinage du pétrole ou est synthétique.

10. Vaseline selon la revendication 9, **caractérisée en ce que** la paraffine synthétique est obtenue par un procédé de Fischer-Tropsch, éventuellement suivi d'une ou plusieurs étapes de traitement choisies parmi un hydroraffinage et un hydrocraquage.

11. Vaseline selon l'une quelconque des revendications 1 à 10, **caractérisée en ce que** la paraffine est conforme à la pharmacopée européenne 7.0 01/2008:1034.

12. Vaseline selon l'une quelconque des revendications 1 à 11, **caractérisée en ce qu'**elle comprend, par rapport au poids total de la vaseline :
- 55 à 70 % en poids d'huile blanche GTL,
- 15 à 30 % en poids de cire micro cristalline, et
- 5 à 15 % en poids de paraffine ;
ou
- 70 à 80 % en poids d'huile blanche GTL,
- 5 à 15 % en poids de cire micro cristalline, et
- 10 à 20 % en poids de paraffine ;
ou
- 50 à 60 % en poids d'huile blanche GTL, et
- 40 à 50 %, en poids de cire microcristalline,
ou
- 40 à 50 % en poids d'huile blanche GTL,
- 10 à 20 % en poids de cire microcristalline, et
- 40 à 50 % en poids de paraffine.

13. Vaseline selon l'une quelconque des revendications 1 à 12, **caractérisée en ce que** la teneur en hydrocarbures aromatiques polycycliques (HAP) de la vaseline est conforme à la pharmacopée européenne 7.0 07/2009:1799.

14. Vaseline selon l'une quelconque des revendications 1 à 13, **caractérisée en ce que** la teneur en matières carbonisables est conforme à la pharmacopée française Codex de 2004.

15. Vaseline selon l'une quelconque des revendications 1 à 14, **caractérisée en ce qu'**elle est conforme à la pharmacopée européenne 7.0 07/2009:1799 et/ou à la pharmacopée française Codex de 2004.

## Patentansprüche

1. Vaseline, umfassend in Bezug auf das Gesamtgewicht der Vaseline:
- 40 bis 90 Gew.-% weißes GTL-Öl,
- 5 bis 50 Gew.-% mikrokristallines Wachs, und
- 0 bis 50 Gew.-% Paraffin.

2. Vaseline nach Anspruch 1, **dadurch gekennzeichnet, dass** das weiße GTL-Öl gemäß einem Fischer-Tropsch-Verfahren zubereitet wird, eventuell gefolgt von einem oder mehreren Verarbeitungsschritten aus einem Hydrocracken, einem Entparaffinieren und einem Hydrofinish.

3. Vaseline nach Anspruch 1 oder 2, **dadurch gekennzeichnet, dass** das weiße GTL-Öl ein C₁₈-C₅₀-Isoparaffin ist.

4. Vaseline nach einem der Ansprüche 1 bis 3, **dadurch gekennzeichnet, dass** der Gehalt an polyzyklischen aromatischen Kohlenwasserstoffen (PAK) des weißen GTL-Öls der europäischen Pharmakopöe 7.0 01/2008:0239 entspricht.

5. Vaseline nach einem der Ansprüche 1 bis 4, **dadurch gekennzeichnet, dass** der Gehalt an leicht carbonisierbaren Substanzen des weißen GTL-Öls der europäischen Pharmakopöe 7.0 01/2008:0239 entspricht.

6. Vaseline nach einem der Ansprüche 1 bis 5, **dadurch gekennzeichnet, dass** das weiße GTL-Öl der europäischen Pharmakopöe 7.0 01/2008:0239 entspricht.

7. Vaseline nach einem der Ansprüche 1 bis 6, **dadurch gekennzeichnet, dass** das mikrokristalline Wachs aus der Raffinierung des Erdöls hervorgegangen ist oder synthetisch ist.

8. Vaseline nach Anspruch 7, **dadurch gekennzeichnet, dass** das synthetische mikrokristalline Wachs ein synthetisches Polymer auf der Basis von Copolymer aus Ethylen und Propylen ist oder ein Wachs, das durch ein Fischer-Tropsch-Verfahren erhalten wird, eventuell gefolgt von einem oder mehreren Verarbeitungsschritten, ausgewählt aus einem Hydroraffinieren, einem Hydrocracken und einer Hydroisomerisierung.

9. Vaseline nach einem der Ansprüche 1 bis 8, **dadurch gekennzeichnet, dass** das Paraffin aus dem Raffinieren des Erdöls hervorgegangen ist oder synthetisch ist.

10. Vaseline nach Anspruch 9, **dadurch gekennzeichnet, dass** das synthetische Paraffin durch ein Fischer-Tropsch-Verfahren erhalten wird, eventuell gefolgt von einem oder mehreren Verarbeitungsschritten, ausgewählt aus einem Hydroraffinieren und einem Hydrocracken.

11. Vaseline nach einem der Ansprüche 1 bis 10, **dadurch gekennzeichnet, dass** das Paraffin der europäischen Pharmakopöe 7.0 01/2008:1034 entspricht.

12. Vaseline nach einem der Ansprüche 1 bis 11, **dadurch gekennzeichnet, dass** sie in Bezug auf das Gesamtgewicht der Vaseline umfasst:
- 55 bis 70 Gew.-% weißes GTL-Öl,
- 15 bis 30 Gew.-% mikrokristallines Wachs, und
- 5 bis 15 Gew.-% Paraffin;
oder
- 70 bis 80 Gew.-% weißes GTL-Öl,
- 5 bis 15 Gew.-% mikrokristallines Wachs, und
- 10 bis 20 Gew.-% Paraffin;
oder
- 50 bis 60 Gew.-% weißes GTL-Öl, und
- 40 bis 50 Gew.-% mikrokristallines Wachs,
oder
- 40 bis 50 Gew.-% weißes GTL-Öl,
- 10 bis 20 Gew.-% mikrokristallines Wachs, und
- 40 bis 50 Gew.-% Paraffin.

13. Vaseline nach einem der Ansprüche 1 bis 12, **dadurch gekennzeichnet, dass** der Gehalt an polyzyklischen aromatischen Kohlenwasserstoffen (PAK) der Vaseline der europäischen Pharmakopöe 7.0 07/2009:1799 entspricht.

14. Vaseline nach einem der Ansprüche 1 bis 13, **dadurch gekennzeichnet, dass** der Gehalt an carbonisierbaren Stoffen der französischen Pharmakopöe Codex von 2004 entspricht.

15. Vaseline nach einem der Ansprüche 1 bis 14, **dadurch gekennzeichnet, dass** sie der europäischen Pharmakopöe 7.0 07/2009:1799 und/oder der französischen Pharmakopöe Codex von 2004 entspricht.

## Claims

1. A petroleum jelly including, in relation to the total weight of petroleum jelly:
- 40 to 90% by weight of white GTL oil,
- 5 to 50% by weight of microcrystalline wax, and
- 0 to 50% by weight of paraffin.

2. The petroleum jelly according to claim 1, **characterized in that** the white GTL oil is prepared by a Fischer-Tropsch process, optionally followed by one or more treatment steps selected from hydrocracking, dewaxing and hydrofinishing.

3. The petroleum jelly according to claim 1 or 2, **characterized in that** the white GTL oil is a C₁₈-C₅₀ isoparaffin.

4. The petroleum jelly according to any one of claims 1 to 3, **characterized in that** the polycyclic aromatic hydrocarbons (PAH) content of the white GTL oil is in accordance with the European Pharmacopoeia 7.0 01/2008:0239.

5. The petroleum jelly according to any one of claims 1 to 4, **characterized in that** the readily carbonizable substances content of the white GTL oil is in accordance with the European Pharmacopoeia 7.0 01/2008:0239.

6. The petroleum jelly according to any one of claims 1 to 5, **characterized in that** the white GTL oil is in accordance with the European Pharmacopoeia 7.0 01/2008:0239.

7. The petroleum jelly according to any one of claims 1 to 6, **characterized in that** the microcrystalline wax results from petroleum refining or is synthetic.

8. The petroleum jelly according to claim 7, **characterized in that** the synthetic microcrystalline wax is a synthetic polymer based on a propylene and ethylene copolymer or a wax obtained by a Fischer-Tropsch process, optionally followed by one or more treatment steps selected from hydrorefining, hydrocracking and hydroisomerization.

9. The petroleum jelly according to any one of claims 1 to 8, **characterized in that** the paraffin results from petroleum refining or is synthetic.

10. The petroleum jelly according to claim 9, **characterized in that** the synthetic paraffin is obtained by a Fischer-Tropsch process, optionally followed by one or more treatment steps selected from hydrorefining and hydrocracking.

11. The petroleum jelly according to any one of claims 1 to 10, **characterized in that** the paraffin is in accordance with the European Pharmacopoeia 7.0 01/2008:1034.

12. The petroleum jelly according to any one of claims 1 to 11, **characterized in that** it includes, in relation to the total weight of petroleum jelly:
- 55 to 70% by weight of white GTL oil,
- 15 to 30% by weight of microcrystalline wax, and
- 5 to 15% by weight of paraffin;
or
- 70 to 80% by weight of white GTL oil,
- 5 to 15% by weight of microcrystalline wax, and
- 10 to 20% by weight of paraffin;
or
- 50 to 60% by weight of white GTL oil, and
- 40 to 50% by weight of microcrystalline wax;
or
- 40 to 50% by weight of white GTL oil,
- 10 to 20% by weight of microcrystalline wax, and
- 40 to 50% by weight of paraffin.

13. The petroleum jelly according to any one of claims 1 to 12, **characterized in that** the polycyclic aromatic hydrocarbons (PAH) content of the petroleum jelly is in accordance with the European Pharmacopoeia 7.0 07/2009: 1799.

14. The petroleum jelly according to any one of claims 1 to 13, **characterized in that** the carbonizable materials content is in accordance with the 2004 French Pharmacopoeia Codex.

15. The petroleum jelly according to any one of claims 1 to 14, **characterized in that** it is in accordance with the European Pharmacopoeia 7.0 07/2009:1799 and/or with the 2004 French Pharmacopoeia Codex.
